# EUROPÄISCHE PATENTSCHRIFT

(11) **EP 1 749 549 B1**
(45) Veröffentlichungstag und Bekanntmachung des Hinweises auf die Patenterteilung: **30.01.2008**
(21) Anmeldenummer: 05016789.9
(22) Anmeldetag: 02.08.2005
(51) Int. Cl.: A61M 27/00, A61B 5/03, A61M 1/00, F04B 43/12

(54) **Liquor-Drainagesystem**
System for drainage of cerebrospinal fluid
Système de drainage de fluide cephalo-rachidien

(43) Veröffentlichungstag der Anmeldung: 07.02.2007
(73) Patentinhaber: Möller Medical GmbH & Co.KG, 36043 Fulda (DE)
(72) Erfinder: Traxler, Christoph, 36093 Künzell (DE); Martens, Daniela, 36043 Fulda (DE); Schröter, Werner, 36137 Grossenlüder (DE); Hölper, Manfred, 83026 Rosenheim (DE); Hölper, Bernd M., 36093 Künzell (DE)
(74) Vertreter: Graf von Stosch, Andreas

(56) Entgegenhaltungen:
- EP-A- 0 982 048
- WO-A-02/07596
- US-A- 4 904 237
- US-A1- 2003 004 495
- US-B1- 6 336 924
- YOON H J ET AL: "Micro devices for a cerebrospinal fluid (CSF) shunt system" 1. Februar 2004 (2004-02-01), SENSORS AND ACTUATORS A, ELSEVIER SEQUOIA S.A., LAUSANNE, CH, PAGE(S) 68-76 , XP004486548 ISSN: 0924-4247 * das ganze Dokument *

## Beschreibung

Die Erfindung bezieht sich allgemein auf eine Vorrichtung zur Drainage von Gehirnflüssigkeit und insbesondere auf ein Liquor-Drainagesystem nach dem Oberbegriff des Anspruchs 1.

Bei Patienten mit Hydrocephalus und/oder erhöhtem Hirndruck wird das überschüssige Gehirnwasser (Liquor cerebrospinalis oder kurz: Liquor) entweder mittels internen Drainagevorrichtungen in eine beliebige Körperhöhle oder ins Blut abgeleitet oder mittels externen Drainagevorrichtungen nach außen drainiert. Eine Liquor-Drainageanordnung besteht im Normalfall aus einem Gehirnkatheter, welcher durch eine Schädelbohrung in die zu behandelnde Gehirnhöhle (Ventrikel) eingeführt und an eine Ablaufleitung nach außen oder zu der gewünschten Körperhöhle bzw. Vene angeschlossen wird.

Die Bildung des Hirnwassers liegt etwa bei 500 ml/Tag, wobei der Druck des Liquors im lumbalen Bereich des Gehirns wie auch in den Gehirnventrikeln einen konstanten Wert innerhalb bestimmter Grenzen aufweist. Bei der Behandlung des Hydrocephalus soll mit Hilfe der Drainagevorrichtung ein reduzierter Druck der Gehirnflüssigkeit im Schädel des Patienten hergestellt werden, wobei der während der Behandlung angestrebte niedrigere Druck der Gehirnflüssigkeit (Liquor-Druck) unterhalb des Hirn-Blutdrucks liegen soll.

Eine externe Liquor-Drainageanordnung ist beispielsweise aus der DE 296 21 904 U1 bekannt, bei der eine sog. Bilanzierungskammer an einem höhenverstellbaren Stativ angebracht ist, in der die drainierte Flüssigkeit gesammelt und deren Volumen bestimmt wird. Aufgrund der Druckempfindlichkeit des Gehirns ist es bei der Drainage von Gehirnflüssigkeit bzw. Liquor außerordentlich wichtig, einen vorgegebenen Drainagedruck einzuhalten. Deshalb muss die Bilanzierungskammer der bekannten Liquor-Drainageanordnung auf eine bestimmte Höhe relativ zum Kopf des Patienten exakt ausgerichtet werden. In der Liquor-Leitung ist ein Druckmesswandler vorgesehen, der den aktuellen Druck der Gehirnflüssigkeit in der Liquor-Leitung misst. Der Drainagedruck und damit der hydrostatische Druck in der Drainageleitung und der Bilanzierungskammer soll dabei bestimmte Grenzwerte nicht über- oder unterschreiten. Wenn die Kopflage des Patienten beispielsweise durch Verschwenken des Bettrückenteils verändert wird, müssen auch die Bilanzierungskammer und der Druckmesswandler in ihrer Höhe verstellt werden, damit einerseits die Druckmessung korrekt ist und andererseits die Druckgrenzwerte eingehalten werden.

Die DE 103 17 308 beschreibt eine Liquor-Drainageanordnung, die ebenfalls an einem höhenverstellbaren Stativ angeordnet ist, mit einer an dem Stativ befestigten Bilanzierungskammer, einer an die Bilanzierungskammer angeschlossenen Liquor-Zulaufleitung, einem Druckmesswandler in der Liquor-Zulaufleitung und einer Peileinrichtung zur Justierung der Höhenlage des Stativs. Bei der in DE 31 27 882 offenbarten Vorrichtung wird zur Steuerung des Vorgangs zum Abfluss des Liquors ein Ventil mit einem bestimmten Schließdruck benutzt. Der Betriebsdruck des in der Ableitungsvorrichtung vorhandenen Ventils entspricht dabei dem gewünschten Liquor-Druck. Der Abfluss der Gehirnflüssigkeit aus den Ventrikeln vollzieht sich so lange, wie der Liquor-Druck über dem Schaltdruck des Ventils liegt. In der DE 693 31 185 ist die Überwachung des Druckes mit optischen Sensormitteln offenbart.

Die Amerikanische Patentschrift US6336924 offenbart ein externes Flüssigkeitsdrainagesystem. Das Drainagesystem umfasst einen zweiteiligen Drainagekatheter. Der erste Teil ist in der Nähe des Patientenkopfs angeordnet und der zweite Teil in der Nähe eines Drainagebeutels. Ein Drucksensor ist zwischen dem ersten und zweiten Teil des Katheters untergebracht. Ein Ende des ersten Teils wird in ein Ventrikel des Patientenkopfes zur Drainage eingeführt. Das andere Ende ist mit dem Drucksensor verbunden. Zur Überwachung der drainierten Flüssigkeit ist ein durchsichtiger Drainagebeutel vorgesehen. Ferner wird vorgeschlagen, mittel eines optischen Tropfenzählsystems die drainierte Flüssigkeit zu erfassen. Eine externe Flusssteuerung ist vorgesehen, um den Fluss durch den Katheter zu steuern. Insbesondere eine Pumpe oder ein steuerbares Ventil werden vorgeschlagen. Die Flusssteuerung wird elektronisch angesteuert, sobald der gemessene Druck einen Schwellwert überschreitet.

Die bekannten Drainagesysteme arbeiten jedoch trotz der druckabhängigen Regelung des Drainageflusses nicht immer zuverlässig und sind relativ teuer.

Es ist daher Aufgabe der vorliegenden Erfindung, ein Liquor-Drainagesystem bereizustellen, welches auf zuverlässige und preiswerte Weise für die Drainage von Gehirnflüssigkeit sorgt.

Die Aufgabe wird durch das erfindungsgemäße Liquor-Drainagesystem gelöst. Das Liquor-Drainagesystem umfasst eine Liquor-Zulaufleitung und einem Drucksensor, der den Druck in der Liquor-Zulaufleitung ermittelt. Eine Pumpe ist vorgesehen, welche die Gehirnflüssigkeit in Abhängigkeit von vorgegebenen Betriebsparametern und gemessenen Betriebsmesswerten abpumpt, Erfassungsmitteln, die das Volumen der drainierten Gehirnflüssigkeit ermitteln. Das Erfassungsmittel ist dazu ausgebildet, das drainierte Liquor-Volumen aus der Rotation der Pumpe und dem Schlauchdurchmesser zu berechnen. Die Pumpe ist dazu ausgebildet, die Gehirnflüssigkeit in Abhängigkeit von dem drainierten Liquor-Volumen und in Abhängigkeit von dem in der Liquor-Zulaufleitung gemessenen Druck abzupumpen.

Die Messung des drainierten Liquorvolumens anhand der Pumprotationen ist preiswert, denn es bedarf keiner weiteren Messvorrichtung zur Ermittlung des drainierten Liquorvolumens. Die kombinierte Verwendung des Drucks und des drainierten Liquor-Volumens zur Steuerung der Pumpe ist zuverlässiger. Denn die alleinige Verwendung des Drucksensors innerhalb der Liquor-Zulaufleitung kann zu Fehlern führen. Die Steuerung der Pumpe in Abhängigkeit von dem drainierten Liquor-Volumen und dem gemessenen Druck erlaubt daher eine genauere und zuverlässigere Regelung des Gehirndrucks.

Gemäß einer bevorzugten Ausführungsform der vorliegenden Erfindung wird ein Liquor-Drainage-Pumpsystem bereitgestellt, das mit einer druckgesteuerten Pumpe gezielt ein bestimmtes Liquor-Volumen abpumpen kann. Da die Liquor-Zulaufleitung bzw. der Gehirnkatheter direkt mit der zu behandelnden Gehirnhöhle (Ventrikel) verbunden ist, herrscht in der Liquor-Zulaufleitung und in der zu behandelnden Gehirnhöhle der gleiche Druck, der durch den in der Liquor-Zulaufleitung angeordneten Drucksensor ermittelt wird. Aus Gründen der Sicherheit und der Genauigkeit der Liquor-Druckmessung ist es auch denkbar, mehrere Drucksensoren vorzusehen, so dass beispielsweise falsche oder fehlende Messwerte eines defekten Drucksensors durch die Messwerte eines intakten Drucksensors ersetzt werden können. Bei mehreren Drucksensoren können deren Messwerte miteinander abgeglichen werden, um die Messgenauigkeit zu überprüfen oder zu verbessern.

Wenn der Gehirndruck bzw. der Druck der Gehirnflüssigkeit (Liquor) über einen definierten oberen Wert steigt, wird die Pumpe vorzugsweise automatisch aktiviert und ein bestimmtes Liquor-Volumen abgepumpt bis sich der gewünschte Druck der Gehirnflüssigkeit eingestellt hat. Dieser Vorgang wird aktiv über den/die in der Liquor-Zulaufleitung installierten Drucksensor(en) überwacht. Sobald ein ausreichendes Volumen von Gehirnflüssigkeit abgepumpt wurde und der Drucksensor in der Liquor-Zulaufleitung feststellt, dass der Gehirndruck wieder auf einen definierten unteren Wert gefallen ist, kann die Pumpe automatisch gestoppt werden.

Der Gehirnwasserdruck kann umso besser gemessen werden, je näher der Drucksensor an der Schädelöffnung angeordnet ist. Gemäß einer weiteren vorteilhaften Ausführungsform der vorliegenden Erfindung ist daher der Drucksensor mit Befestigungsmitteln ausgestattet, die eine Anordnung des Drucksensors im Kopfbereich des Patienten ermöglichen. So kann der Drucksensor beispielsweise über ein Stirnband in der Nähe der Schädelbohrung befestigt werden oder über einen Ohrbügel oder Klipp am Ohr des Patienten angeordnet werden.

Die Steuerung des Liquor-Drainagesystems erfolgt zweckmäßigerweise durch eine elektronische Steuerung. In der elektronischen Steuerung werden die für den Betrieb der Pumpe erforderlichen Berechnungen aufgrund der vorgegebenen Betriebsparameter und der Betriebsmesswerte bzw. der gemessenen Betriebsdaten vorgenommen. Die elektronische Steuerung des Liquor-Drainagesystems ist vorzugsweise programmierbar, so dass über eine entsprechende Software bestimmte Betriebsabläufe beispielsweise für unterschiedliche medizinische Anwendungen vorgenommen werden können. Ferner können in der elektronischen Steuerung des Liquor-Drainagesystems Alarmfunktionen eingerichtet sein, die bei einer Abweichung der gemessenen Betriebsdaten von einem vorgegebenen Bereich für den Druck oder das Pumpvolumen durch optische und/oder akustische Mittel Alarm geben. Ein solches Alarmsignal wird zweckmäßigerweise über eine entsprechende Schnittstelle auch elektronisch an eine externe Überwachung weitergegeben.

Gemäß einer bevorzugten Ausführungsform der vorliegenden Erfindung umfasst das Liquor-Drainagesystem Eingabemittel, über die bestimmte Betriebsparameter, wie z.B. vorgegebene Druck-Grenzwerte des Liquor-Drucks bzw. des Gehirndrucks in dem zu behandelnden Ventrikel, in die Steuerungselektronik eingegeben werden können. In der Steuerungselektronik können auch elektronische Speichermittel vorgesehen sein, in denen beispielsweise bestimmte Betriebsabläufe des Liquor-Drainagesystems abgespeichert bzw. vorgehalten werden können. Das Liquor-Drainagesystem kann ferner Bedienungselemente umfassen, über die der Betrieb der Pumpe unmittelbar gesteuert werden kann. Dadurch ist die Möglichkeit gegeben, die Pumpe auch manuell zu steuern, beispielsweise um zur Beseitigung von Verstopfungen kurzfristig eine höhere Pumpleistung zu erzeugen.

Der Drucksensor in der Liquor-Zulaufleitung ist vorzugsweise als elektronischer Drucksensor ausgebildet, der den in der Liquor-Zulaufleitung gemessenen Druck in Form von elektrischen Signalen an die Steuerungselektronik weiterleitet. Aufgrund der vom Drucksensor gelieferten Drucksignale und der vorgegebenen Druck-Grenzwerte berechnet die Steuerungselektronik den erforderlichen Betrieb des Liquor-Drainagesystems bzw. den Betrieb der Liquor-Pumpe. Dabei führt die Steuerungselektronik insbesondere einen Vergleich zwischen den vorgegebenen Liquor-Druckgrenzwerten und dem vom Drucksensor ermittelten Druck in der Liquor-Zulaufleitung durch. Die Steuerungselektronik entscheidet aufgrund der Berechnung, ob die Pumpe des Liquor-Drainagesystems aktiviert oder deaktiviert werden soll und/oder mit welcher Pumpleistung und ggf. über welche Zeitspanne die Liquor-Drainagepumpe betrieben werden soll.

Als Pumpe wird zweckmäßigerweise eine Schlauchpumpe verwendet, die den Vorzug hat, dass die gepumpte Flüssigkeit innerhalb des Schlauchssystems nicht mit irgendwelchen Fremdkörpern in Verbindung kommt. Als Drucksensor ist ein Piezo-Drucksensor besonders geeignet, der auch minimale Druckveränderungen mit Hilfe piezokeramischer Materialien ermitteln kann und als elektrische Impulse abgibt.

Zur Verbindung der einzelnen flüssigkeitsführenden Komponenten ist das erfindungsgemäße Liquor-Drainagesystem mit einem Schlauchsystem mit entsprechenden Verzweigungen ausgestattet. Dieses Schlauchsystem umfasst eine Liquor-Zulaufleitung bzw. einen Gehirnkatheter, der von dem Kopf des Patienten zur Pumpe führt, sowie eine Liquor-Ablaufleitung, die von der Pumpe zu einem Auffangbeutel zum Auffangen des drainierten Liquors führt.

Im Gegensatz zu den aus dem Stand der Technik bekannten Liquor-Drainageanordnungen, bei denen Liquor nur durch seinen hydrostatischen Eigendruck in einen Auffangbehälter abläuft, wird bei dem erfindungsgemäßen System der Liquor gezielt abgepumpt, wobei das abgepumpte Volumen druckgesteuert geregelt wird. Auf diese Weise ist es möglich, die Liquor-Volumina und die intrakraniellen Druckwerte, über die der Pumpvorgang geregelt wird, frei einzustellen. Zusätzlich ist auch die Generierung eines Druckes im Schlauchsystem unterhalb einer definierten Untergrenze möglich, um z.B. die Durchgängigkeit des Schlauchsystems zu überprüfen oder das Schlauchsystem verschließende Fremdkörper (z.B. Blutkoagel) zu lösen. Dadurch kann mit dem erfindungsgemäßen Liquor-Drainagesystem auch eine rein volumengesteuerte Liquor-Drainage, wie sie z.B. für lumbale Dauerdrainagen notwendig ist, durchgeführt werden.

Bei dem Schlauchsystem des erfindungsgemäßen Liquor-Drainagesystems handelt es sich um ein geschlossenes Schlauchsystem, das eine gleichzeitige Messung des Drucks über den Drucksensor während des Pumpvorgangs ermöglicht. Dadurch können mittels des Drucksensors z.B. Diskonnektionen der Schlauchverbindungen aufgrund von akutem Druckabfall im Schlauchsystem oder Schlauchokklusionen bzw. Schlauchverschlüsse durch sehr niedrige oder sehr hohe Druckwerte während des Pumpvorganges erfasst werden.

Gemäß einer weiteren bevorzugten Ausführungsform der vorliegenden Erfindung werden die Betriebsdaten des Liquor-Drainagesystems kontinuierlich oder in Intervallen erfasst. Beispielsweise kann das Liquor-Drainagevolumen aus der Rotation der Pumpe und dem Schlauchdurchmesser kontinuierlich berechnet werden. Dabei kann eine Aufzeichnung und Dokumentation des zeitlichen Verlaufes des ermittelten Pumpvolumens und des Drucks in der Liquor-Zulaufleitung erfolgen. Diese Daten können zur weiteren Auswertung von der Steuerungselektronik des Liquor-Drainagesystems an eine Auswerteeinheit weitergeleitet werden. Dadurch wird eine automatisierte Auswertung des zeitlichen Hirndruck- und Liquor-Drainageverlaufs ermöglicht, was bei bisherigen Systemen nur durch Ablesen des Flüssigkeitsstands am Auffangbeutel möglich war.

Die Betriebsdaten des Liquor-Drainagesystems, wie z.B. die Aufzeichnung und Dokumentation des zeitlichen Verlaufes des ermittelten Pumpvolumens oder des Drucks in der Liquor-Zulaufleitung, die Laufzeit der Pumpe, das von der Pumpe geförderte Gesamtvolumen, der Betriebsmodus oder die Laufzeit der Pumpe können zweckmäßig in den elektronischen Speichermitteln des erfindungsgemäßen Liquor-Drainagesystems gespeichert und abgerufen werden. Zur Gewährleistung der Datensicherheit können Eingabemittel vorgesehen sein, mit denen die in den Speichermitteln des Liquor-Drainagesystems abgelegten Daten manuell gelöscht werden. Das erfindungsgemäße Liquor-Drainagesystem verfügt zweckmäßigerweise über eine optische Anzeige, über die eingestellte Betriebsparameter und/oder aktuelle Messwerte von Betriebsdaten angezeigt werden können.

Die Datenübertragung vom Liquor-Drainagesystem an eine externe Auswerteeinheit erfolgt über eine Schnittstelle, wie z.B. eine Kabel-, Infrarot-, bzw. Funkverbindung oder mit Hilfe einer Speicherkarte/Chip. Das erfindungsgemäße Liquor-Drainagesystem kann ferner mit einem zusätzlichen Ausgang zur Übertragung von Betriebsdaten, insbesondere des Liquor-Drucksignals an externe Anzeigemonitore sowie mit einem Rufknopf für das Pflegepersonal ausgestattet sein. Um einen netztunabhängigen Betrieb (beispielsweise bei Stromausfällen) zu gewährleisten ist das Liquor-Drainagesystem vorzugsweise mit einem Speicher für elektrische Energie bzw. einem Akkumulator ausgestattet, der den netztunabhängigen Betrieb für mindestens einige Stunden sicherstellt und einen Transport des Patienten mit dem System erleichtert.

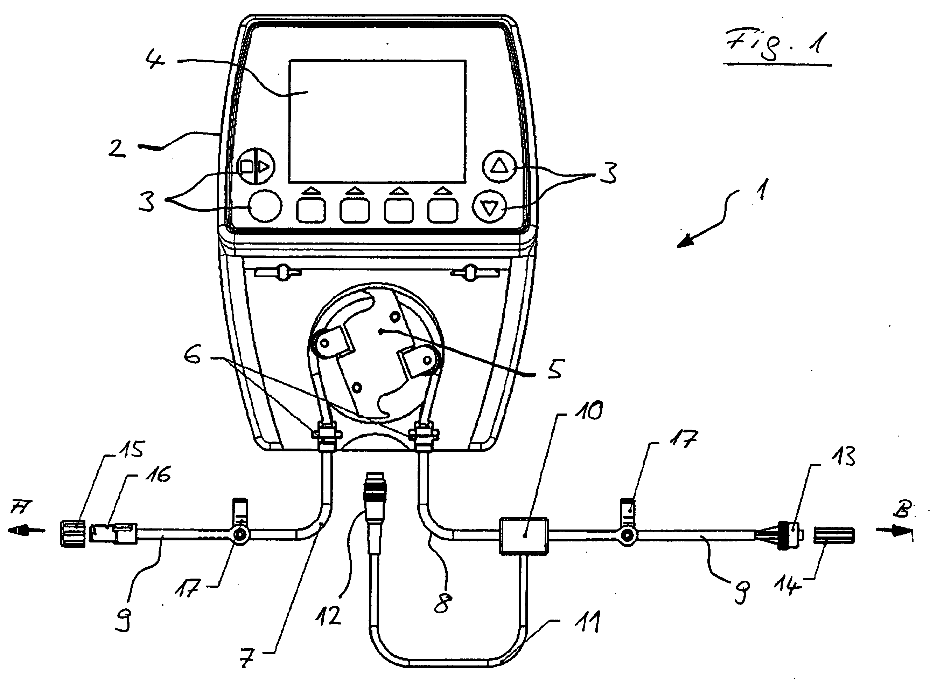

Weitere Einzelheiten, bevorzugte Ausführungsformen und Vorteile der vorliegenden Erfindung ergeben sich aus der nachfolgenden Beschreibung unter Bezugnahme auf die Zeichnung. Figur 1 zeigt eine Skizze vom Aufbau eines Liquor-Drainagesystems gemäß einer bevorzugten Ausführungsform der vorliegenden Erfindung.

Die in Fig. 1 gezeigte Ausführungsform des Liquor-Drainagesystems 1 umfasst nach der vorliegenden Erfindung eine Hauptkomponente 2 mit einem Gehäuse 2, in dem eine elektronische Steuerung mit elektronischen Mitteln für die oben beschriebenen Berechnungen untergebracht ist. An dem Gehäuse 2 sind Eingabe- und Bedienungselemente 3 angeordnet, über die für den Betrieb des Liquor-Drainagesystems 1 erforderliche Betriebsparameter oder ein gewünschter Betriebsmodus des Liquor-Drainagesystems 1 manuell eingegeben werden können. Über eine Anzeige 4 können sämtliche eingegebenen Betriebsparameter und/oder aktuelle Betriebsmesswerte und Betriebsmodi des Liquor-Drainagesystems 1 angezeigt werden.

Die Hauptkomponente 2 des Liquor-Drainagesystems 1 umfasst ferner eine Schlauchpumpe 5 mit Anschlüssen 6 für Schlauchverbindungen 9 eines Schlauchsystems. Bei den an die Schlauchpumpe 5 angeschlossenen Schlauchverbindungen 9 handelt es sich einerseits um eine Liquor-Zulaufleitung (Gehirnkatheter) 8 zwischen der Schlauchpumpe 5 und dem Patienten und andererseits um eine Schlauchverbindung 7 zwischen der Schlauchpumpe 5 und einem Ableitbeutel (nicht gezeigt), in dem sich die abgepumpte Gehirnflüssigkeit (Liquor) sammelt. In der Figur ist durch den Pfeil A die Anschlussrichtung der Schlauchverbindung 7 zum Ableitbeutel und durch den Pfeil B die Anschlussrichtung der Schlauchverbindung 8 zum Patienten angedeutet. Es hat sich als vorteilhaft erwiesen, wenn die Schlauchverbindungen 9 des Schlauchsystems einen Innendurchmesser von etwa 1,0 mm, einen Außendurchmesser von etwa 4,0 mm, eine Wandstärke von etwa 1,5 mm und eine Härte in dem Bereich von 50-55 Shore A haben.

Die Schlauchpumpe 5 ist in bekannter Weise aufgebaut und umfasst ein flexibles kreisförmig angeordnetes Pumpschlauchsegment. Im Mittelpunkt des kreisförmig angeordneten Pumpschlauchsegments ist die Achse eines Pumpenrotors gelagert, an dessen radial äußeren Enden drehbar gelagerte Rollen vorgesehen sind. Mit der Drehung des Pumpenrotors wälzen sich die Rollen auf dem kreisförmigen Pumpschlauchsegment ab, wobei das Pumpschlauchsegment zusammengedrückt wird. Durch die Drehbewegung des Pumpenrotors mit den auf dem Pumpschlauchsegment abwälzenden Rollen wird ein in dem Pumpschlauchsegment befindliches Fluid in Drehrichtung des Pumpenrotors befördert. Eine solche Schlauchpumpe hat den Vorteil, dass das in dem Schlauch befindliche Fluid nicht mit Fremdkörpern in Kontakt kommt und damit eine Verunreinigung des Fluids ausgeschlossen ist.

An der Schlauchverbindung (Gehirnkatheter bzw. Liquor-Zulaufleitung) 8 zwischen der Schlauchpumpe 5 und dem Patienten ist ein Drucksensor 10 angeordnet, der den Druck des Liquors in der Liquor-Zulaufleitung 8 permanent oder in Intervallen misst. Die von dem Drucksensor ermittelten Druckmesswerte werden in elektrische Signale umgewandelt und über eine elektrische Leitung 11 an die elektrische Steuerung in der Hauptkomponente 2 des Liquor-Drainagesystems 1 weitergeleitet. Dazu wird die elektrische Leitung 11 vom Drucksensor 10 über einen Anschluss 12 an die Hauptkomponente 2 des Liquor-Drainagesystems 1 über eine entsprechende Schnittstelle (nicht dargestellt) angeschlossen.

Die elektrische Steuerung des Liquor-Drainagesystems 1 verwendet die von dem Drucksensor 10 übermittelten Betriebsmesswerte vom Druck in der Liquor-Zulaufleitung 8 als Grundlage für die Berechnungen in der elektrischen Steuerung zum geregelten Betrieb der Schlauchpumpe 5. Wenn über den Drucksensor 10 beispielsweise ein Druck in der Liquor-Zulaufleitung 8 festgestellt wird, der oberhalb eines vorgegebenen Betriebsparameters oder außerhalb eines bestimmten Druckbereichs liegt, kann die elektrische Steuerung des Liquor-Drainagesystems 1 die Schlauchpumpe 5 aktivieren.

Die elektrische Steuerung überwacht während des Pumpvorgangs über den Drucksensor 10 kontinuierlich den Druck in der Liquor-Zulaufleitung 8. Sobald der Druck in der Liquor-Zulaufleitung 8 einen vorgegebenen Betriebsparameter erreicht hat oder einen bestimmten unteren Grenzwert des Drucks in der Liquor-Zulaufleitung 8 erreicht bzw. unterschritten hat, kann die elektrische Steuerung den Betrieb der Schlauchpumpe 5 einstellen. Zusätzlich kann das Liquor-Drainagesystem 1 Erfassungsmittel beinhalten, die das geförderte Volumen des Liquors ermitteln und das drainierte Liquor-Volumen als Grundlage für die Steuerung der Pumpe 5 verwenden. Dazu kann beispielsweise die Anzahl der Umdrehungen des Pumpenrotors herangezogen werden, da diese in einem direktem Verhältnis zu den von der Schlauchpumpe 5 geförderten Volumen steht.

Sowohl die Schlauchverbindung 7 zwischen der Schlauchpumpe 5 und dem Ableitbeutel als auch die Liquor-Zulaufleitung 8 zwischen der Schlauchpumpe und dem Patienten können mit einer Verzweigung beispielsweise in Form eines 3-Wege-Hahns 17 ausgestattet sein, über die weitere Schlauchleitungen an das Schlauchsystem angeschlossen werden können. Die Schlauchverbindungen 9 werden jeweils über geeignete Schlauchanschlusselemente 13, 14, 15, 16 an weitere Schlauchsegmente oder andere Liquor-Flüssigkeit führende Komponenten des Liquor-Drainagesystems 1 angeschlossen.

Die Schlauchanschlusselemente bzw. Schlauchkupplungen 13, 14, 15, 16 sind jeweils so ausgebildet, dass sie einen drucksicheren Anschluss der Schlauchverbindungen und damit ein abgeschlossenes Schlauchsystem gewährleisten. Zusätzlich können die Schlauchanschlusselemente bzw. Schlauchkupplungen 13, 14, 15, 16 so ausgebildet sein, dass sie eine Einmalverwendbarkeit der Schlauchverbindungen 7, 8 bzw. des gesamten Schlauchsets sicherstellen, d.h. dass eine Schlauchverbindungen 7, 8 nur einmal in dem abgeschlossenen Schlauchsystem verwendet werden können, um deren Sterilität zu gewährleisten. Ferner kann zumindest ein Anschlusselement 13, 14, 15, 16 so ausgebildet sein, dass der Drucksensor 10 darin aufgenommen bzw. integriert werden kann. Auf diese Weise wäre der Drucksensor in unmittelbarem Kontakt mit dem Liquor im Drainagesystem untergebracht und leicht austauschbar montiert.

Auf diese Weise kann sichergestellt werden, dass der vom Drucksensor 10 gemessene Druck in der Liquor-Zulaufleitung 8 an der Messstelle auch dem Druck in der behandelten Gehirnhöhle entspricht. Das erfindungsgemäße Liquor-Drainagesystem 1 zeichnet sich einerseits durch einfache Bedienbarkeit aus und gewährleistet andererseits einen kontrollierten Liquor-Drainagevorgang, bei dem das drainierte Liquor-Volumen je nach Bedarf geregelt und der Drainagedruck bzw. der Liquor-Druck zuverlässig innerhalb eines bestimmten Druckbereichs gehalten werden kann.

### Liste der Bezugszeichen

- 1: Liquor-Drainagesystem
- 2: Gehäuse der Hauptkomponente des Liquor-Drainagesystems 1
- 3: Eingabe- bzw. Bedienungselemente
- 4: Anzeige
- 5: Schlauchpumpe
- 6: Schlauchanschlüsse an die Schlauchpumpe 5
- 7: Schlauchverbindung zwischen Schlauchpumpe 5 und Ableitbeutel
- 8: Schlauchverbindung zum Patienten bzw. Liquor-Zulaufleitung
- 9: Schlauchteile eines geschlossenen Schlauchsystems
- 10: Drucksensor
- 11: elektrische Leitung vom Drucksensor 10 zur elektronischen Steuerung
- 12: elektrischer Anschluss der Leitung 11 an die elektronische Steuerung
- 13: Schlauchanschlusselement
- 14: Schlauchanschlusselement
- 15: Schlauchanschlusselement
- 16: Schlauchanschlusselement
- 17: Dreiwegehahn
- A: Anschlussrichtung zum Ableitbeutel
- B: Anschlussrichtung zum Patienten

## Patentansprüche

1. Liquor-Drainagesystem (1) zur Drainage von Gehirnflüssigkeit (Liquor) mit
einer Liquor-Zulaufleitung (8) und einem Drucksensor (10), der den Druck in der Liquor-Zulaufleitung (8) ermittelt, und
einer Pumpe (5), welche die Gehirnflüssigkeit in Abhängigkeit von vorgegebenen Betriebsparametern und gemessenen Betriebsmesswerten abpumpt, Erfassungsmitteln, die das Volumen der drainierten Gehirnflüssigkeit ermitteln,
**dadurch gekennzeichnet, dass**
das Erfassungsmittel dazu ausgebildet ist, das drainierte Liquor-Volumen aus der Rotation der Pumpe und dem Schlauchdurchmesser zu berechnen, die Pumpe (5) dazu ausgebildet ist, die Gehirnflüssigkeit in Abhängigkeit von dem drainierten Liquor-Volumen und in Abhängigkeit von dem in der Liquor-Zulaufleitung (8) gemessenen Druck abzupumpen.

2. System gemäß Anspruch 1, wobei die vorgegebenen Betriebsparameter und die Betriebsmesswerte zumindest den in der Liquor-Zulaufleitung (8) gemessenen Druck oder das abgepumpte Liquor-Volumen umfassen.

3. System gemäß einem der vorangehenden Ansprüche, wobei die Pumpe (5) als Schlauchpumpe ausgebildet ist.

4. System gemäß einem der vorangehenden Ansprüche, wobei der Drucksensor (10) an der Liquor-Zulaufleitung (8), vorzugsweise nahe dem Patienten angeordnet ist.

5. System gemäß einem der vorangehenden Ansprüche, wobei der Drucksensor (10) mit Befestigungsmitteln versehen ist, die eine Anordnung des Drucksensors (10) im Kopfbereich des Patienten, vorzugsweise am Ohr des Patienten ermöglichen.

6. System gemäß einem der vorangehenden Ansprüche, wobei mehrere Drucksensoren (10) vorgesehen sind, die gemeinsam oder alternativ den Druck in der Liquor-Zulaufleitung (8) ermitteln.

7. System gemäß einem vorangehenden der Ansprüche, wobei der Drucksensor (10) als elektronischer Drucksensor, insbesondere als Piezo-Drucksensor ausgebildet ist, der Druckveränderungen in der Liquor-Zulaufleitung (8) in elektrische Impulse umwandelt.

8. System gemäß einem der vorangehenden Ansprüche, wobei das Liquor-Drainagesystem (1) eine elektronische Steuerung umfasst, welche die für den Betrieb der Pumpe (5) erforderlichen Berechnungen aufgrund der vorgegebenen Betriebsparameter und der gemessenen Betriebsmesswerte durchführt.

9. System gemäß Anspruch 8, wobei die elektronische Steuerung programmierbar ist, so dass bestimmte Betriebsabläufe, z.B. für unterschiedliche medizinische Anwendungen durchführbar sind.

10. System gemäß einem der Ansprüche 8 oder 9, wobei die elektronische Steuerung Alarmfunktionen umfasst, die bei einer Abweichung eines gemessenen Betriebsmesswerts von dem entsprechenden vorgegebenen Betriebsparameter aktiviert werden.

11. System gemäß einem der Ansprüche 8 bis 10, wobei die elektronische Steuerung mindestens eine Schnittstelle umfasst, wie z.B. eine Kabel-, Infrarot-, bzw. Funkverbindung oder eine entnehmbare Speicherkarte/Chip, über die Betriebsmesswerte und/oder Betriebsparameter an ein externes Überwachungssystem oder eine externe Anzeigevorrichtung weitergegeben werden können.

12. System gemäß einem der Ansprüche 8 bis 11, wobei die elektronische Steuerung elektronische Speichermittel umfasst, in denen z.B. vorgegebene Betriebsabläufe des Liquor-Drainagesystems (1) oder eine zeitliche Dokumentation vom Betrieb des Liquor-Drainagesystems (1) abgespeichert werden können.

13. System gemäß einem der vorangehenden Ansprüche, wobei das Liquor-Drainagesystem (1) Eingabemittel aufweist, über die Betriebsparameter, wie z.B. vorgegebene Druck-Grenzwerte des Liquor-Drucks in die elektronische Steuerung eingegeben werden können.

14. System gemäß einem der vorangehenden Ansprüche, wobei das Liquor-Drainagesystem (1) Bedienungselemente aufweist, über die z.B. der Betrieb der Pumpe (5) unmittelbar steuerbar ist oder eine Alarmfunktion manuell aktivierbar ist.

15. System gemäß einem der vorangehenden Ansprüche, wobei das Liquor-Drainagesystem (1) eine optische Anzeige aufweist, über die eingestellte Betriebsparameter und/oder aktuelle Betriebsmesswerte angezeigt werden können.

16. System gemäß einem der vorangehenden Ansprüche, wobei das Liquor-Drainagesystem (1) mit einem Speicher für elektrische Energie bzw. einem Akkumulator zur netzunabhängigen Stromversorgung ausgestattet ist.

17. System gemäß einem der vorangehenden Ansprüche, wobei das Liquor-Drainagesystem (1) ein abgeschlossenes Schlauchsystem (9) mit abgeschlossenen Schlauchverbindungen (7, 8) zur Verbindung der flüssigkeitsführenden Komponenten umfasst.

18. System gemäß Anspruch 17, wobei das abgeschlossene Schlauchsystem (9) Mittel umfasst, insbesondere Anschlusselemente (13, 14, 15, 16), die eine Einmalverwendbarkeit der Schlauchverbindungen (7, 8) sicherstellen.

19. System gemäß Anspruch 18, wobei der Drucksensor (10) in einem der Anschlusselemente (13, 14, 15, 16) integriert ist.

20. System gemäß einem der vorangehenden Ansprüche, wobei das Liquor-Drainagesystem (1) einen Ableitbeutel zum Auffangen der abgeleiteten Gehirnflüssigkeit umfasst.

## Claims

1. Liquor drainage system (1) for the drainage of cerebrospinal fluid (liquor), with a liquor feed line (8) and a pressure sensor (10), which determines the pressure in the liquor feed line (8), and
a pump (5) which pumps out the cerebrospinal fluid as a function of preset operating parameters and measured operating measured values, detection means which determine the volume of the drained cerebrospinal fluid,
**characterized in that**
detection means is constructed to calculate the drained volume of liquor from the rotation of the pump and the hose diameter, the pump (5) is constructed to pump out the cerebrospinal fluid as a function of drained volume of liquor and as a function of the pressure measured in the liquor feed line (8).

2. System according to claim 1, wherein the operating parameters und the operating measured values comprise at least the pressure measured in the liquor feed line (8) or the volume of liquor pumped out.

3. System according to one of the preceding claims, wherein the pump (5) is constructed as a hose pump.

4. System according to one of the preceding claims, wherein the pressure sensor (10) on the liquor feed line (8) is preferably arranged near the patient.

5. System according to one of the preceding claims, wherein the pressure sensor (10) is provided with fastening means which enable the pressure sensor (10) to be arranged in the region of the patient's head, preferably on the patient's ear.

6. System according to one of the preceding claims, wherein several pressure sensors (10) are provided, which jointly or alternatively determine the pressure in the liquor feed line (8).

7. System according to one of the preceding claims, wherein the pressure sensor (10) is constructed as an electronic pressure sensor, in particular as a piezoresistive pressure sensor which converts changes in pressure in the liquor feed line (8) into electric pulses.

8. System according to one of the preceding claims, wherein the liquor drainage system (1) comprises an electronic control unit which carries out calculations required for the operation of the pump (5) on the basis of the preset operating parameters and the measured operating measured values.

9. System according to claim 8, wherein the electronic control unit is programmable in such a way that specific operating cycles, e.g. for different medical applications, can be carried out.

10. System according to one of claims 8 or 9, wherein the electronic control unit comprises alarm functions which are activated if there is a deviation of a measured operating measured value from the corresponding preset operating parameter.

11. System according to one of claims 8 to 10, wherein the electronic control unit comprises at least one interface, such as, e.g. a cable, infrared or radio connection or a removable memory card/chip, via which operating measured values and/or operating parameters can be transmitted to an external monitoring system or an external display device.

12. System according to one of claims 8 to 11, wherein the electronic control unit comprises electronic memory means, in which, e.g. preset operating cycles of the liquor drainage system (1) or chronological documentation of the operation of the liquor drainage system (1) can be stored.

13. System according to one of the preceding claims, wherein the liquor drainage system (1) has input means via which operating parameters, such as, e.g. preset pressure limit values of the liquor pressure can be input into the electronic control unit.

14. System according to one of the preceding claims, wherein the liquor drainage system (1) has operating elements via which, e.g. the operation of the pump (5) can be directly controlled or an alarm function can be manually activated.

15. System according to one of the preceding claims, wherein the liquor drainage system (1) has an optical display, via which set operating parameters and/or current operating measured values can be displayed.

16. System according to one of the preceding claims, wherein the liquor drainage system (1) is equipped with a store for electric energy or an accumulator for mainsindependent power supply.

17. System according to one of the preceding claims, wherein the liquor drainage system (1) comprises a closed hose system (9) with closed hose connections (7, 8) for connecting the fluid-carrying components.

18. System according to claim 17, wherein the closed hose system (9) comprises means, in particular connecting elements (13, 14, 15, 16) which ensure that the hose connections (7, 8) can be used once only.

19. System according to claim 18, wherein the pressure sensor (10) is integrated into one of the connecting elements (13, 14, 15, 16).

20. System according to one of the preceding claims, wherein the liquor drainage system (1) comprises a drainage bag for collecting the drained cerebrospinal fluid.

## Revendications

1. Système de drainage du liquide céphalo-rachidien (1) destiné à drainer le liquide cérébro-spinal (liquide céphalo-rachidien), muni
d'une conduite d'arrivée (8) du liquide céphalo-rachidien et d'un capteur de pression (10), qui détermine la pression dans la conduite d'arrivée (8) du liquide céphalo-rachidien, et
d'une pompe (5), qui évacue par pompage le liquide cérébro-spinal en fonction de paramètres de fonctionnement prédéterminés et de valeurs de fonctionnement mesurées, de moyens de détection qui déterminent le volume de liquide cérébro-spinal drainé,
**caractérisé en ce que**
le moyen de détection est conçu pour calculer le volume du liquide céphalo-rachidien drainé à partir de la rotation de la pompe et du diamètre de tuyau flexible, la pompe (5) est conçue pour évacuer par pompage le liquide cérébro-spinal en fonction du volume de liquide céphalo-rachidien drainé et en fonction de la pression mesurée dans la conduite d'arrivée (8) du liquide céphalo-rachidien.

2. Système selon la revendication 1, les paramètres de fonctionnement prédéterminés et les valeurs de fonctionnement mesurées comprenant au moins la pression mesurée dans la conduite d'arrivée (8) du liquide céphalo-rachidien ou le volume du liquide céphalo-rachidien évacué par pompage.

3. Système selon l'une des revendications précédentes, la pompe (5) étant conçue comme pompe péristaltique.

4. Système selon l'une des revendications précédentes, le capteur de pression (10) étant disposé sur la conduite d'arrivée (8) du liquide céphalo-rachidien, de préférence à proximité du patient.

5. Système selon l'une des revendications précédentes, le capteur de pression (10) étant pourvu de moyens de fixation qui permettent un agencement du capteur de pression (10) dans la région de tête du patient, de préférence au niveau de l'oreille du patient.

6. Système selon l'une des revendications précédentes, plusieurs capteurs de pression (10) étant prévus qui déterminent en commun ou en alternance la pression dans la conduite d'arrivée (8) du liquide céphalo-rachidien.

7. Système selon l'une des revendications précédentes, le capteur de pression (10) étant conçu comme capteur de pression électronique, en particulier comme capteur de pression piézoélectrique, qui convertit en impulsions électriques les variations de pression dans la conduite d'arrivée (8) du liquide céphalo-rachidien.

8. Système selon l'une des revendications précédentes, le système de drainage du liquide céphalo-rachidien (1) comprenant une commande électronique, qui effectue les calculs nécessaires au fonctionnement de la pompe (5) à partir des paramètres de fonctionnement prédéterminés et des valeurs de fonctionnement mesurées.

9. Système selon la revendication 8, la commande électronique étant programmable, de sorte que certains déroulements opératoires, par exemple pour différentes applications médicales, sont réalisables.

10. Système selon l'une des revendications 8 ou 9, la commande électronique comprenant des fonctions d'alarme, qui sont activées lorsqu'une valeur de fonctionnement mesurée s'écarte du paramètre de fonctionnement prédéterminé correspondant.

11. Système selon l'une des revendications 8 à 10, la commande électronique comprenant au moins une interface, comme par exemple une liaison câblée, infrarouge ou radio ou une puce/carte à mémoire amovible, par l'intermédiaire de laquelle les valeurs de fonctionnement mesurées et/ou les paramètres de fonctionnement peuvent être transmis à un système externe de contrôle ou à un dispositif externe d'affichage.

12. Système selon l'une des revendications 8 à 11, la commande électronique comprenant des moyens électroniques de mémorisation dans lesquels peuvent être mémorisés par exemple des déroulements opératoires prédéterminés du système de drainage du liquide céphalo-rachidien (1) ou une documentation temporelle du fonctionnement du système de drainage du liquide céphalo-rachidien (1).

13. Système selon l'une des revendications précédentes, le système de drainage du liquide céphalo-rachidien (1) comportant des moyens d'entrée, par l'intermédiaire desquels des paramètres de fonctionnement, comme par exemple les valeurs limites prédéterminées de la pression du liquide céphalo-rachidien, peuvent être entrés dans la commande électronique.

14. Système selon l'une des revendications précédentes, le système de drainage du liquide céphalo-rachidien (1) comportant des éléments de commande par l'intermédiaire desquels par exemple le fonctionnement de la pompe (5) peut être commandé directement ou une fonction d'alarme peut être activée manuellement.

15. Système selon l'une des revendications précédentes, le système de drainage du liquide céphalo-rachidien (1) comportant un affichage optique par l'intermédiaire duquel des paramètres de fonctionnement réglés et/ou les valeurs de fonctionnement mesurées actuelles peuvent être affichés.

16. Système selon l'une des revendications précédentes, le système de drainage du liquide céphalo-rachidien (1) étant équipé d'un réservoir pour l'énergie électrique et/ou d'un accumulateur pour l'alimentation électrique indépendante du secteur.

17. Système selon l'une des revendications précédentes, le système de drainage du liquide céphalo-rachidien (1) comprenant un système fermé de tuyaux flexibles (9) comportant des liaisons à tuyaux flexibles (7, 8) afin de relier les composants conduisant le liquide.

18. Système selon la revendication 17, le système fermé de tuyaux flexibles (9) comprenant des moyens, en particulier des éléments de raccordement (13, 14, 15, 16) qui garantissent la possibilité d'usage unique des liaisons à tuyaux flexibles (7, 8).

19. Système selon la revendication 18, le capteur de pression (10) étant intégré dans un des éléments de liaison (13, 14, 15, 16).

20. Système selon l'une des revendications précédentes, le système de drainage du liquide céphalo-rachidien (1) comprenant une poche d'évacuation destinée à recueillir le liquide céphalo-rachidien évacué.
